# EUROPEAN PATENT SPECIFICATION

(11) **EP 3 688 889 B1**
(45) Date of publication and mention of the grant of the patent: **26.05.2021**
(21) Application number: 19711142.0
(22) Date of filing: 22.03.2019
(51) Int. Cl.: H04B 10/00, H04B 5/00, G02B 6/36, H01P 1/06, A61B 6/03

(54) **ROTARY JOINT WITH DIELECTRIC WAVEGUIDE**
DREHVERBINDUNG MIT DIELEKTRISCHEM WELLENLEITER
JOINT ROTATIF À GUIDE D'ONDES DIÉLECTRIQUE

(30) Priority: 22.03.2018 EP 18163449
(43) Date of publication of application: 05.08.2020
(73) Proprietor: Schleifring GmbH, 82256 Fürstenfeldbruck (DE)
(72) Inventor: KRUMME, Nils, 82340 Feldafing (DE); STEFFENS, Holger, 80997 München (DE)
(74) Representative: Lohr, Jöstingmeier & Partner
(86) International application number: PCT/EP2019/057262
(87) International publication number: WO 2019/180215

(56) References cited:
- EP-A1- 2 932 901
- US-A- 4 692 721
- US-A1- 2007 063 785
- US-A1- 2017 332 991

## Description

### Field of the invention

The invention relates to high speed datalinks for non-contacting or contactless signal and data transmission, in particular to rotating transmission devices like rotary joints. Such transmission devices may be used in computer tomography scanners, also called CT scanners. These datalinks may be used in a mm-wave range.

### Description of the related art

Contactless rotating couplers, also called contactless rotary joints are used to couple signals and data between parts rotating against each other. For example, in CT scanners, a rotating x-ray tube and an x-ray detector generate high-speed imaging data. The data may be transmitted from the rotating part to the station-any part. Furthermore, control signals for controlling the device and specifically the power supply of the x-ray tube may be transmitted from the stationary to the rotating part and vice versa. Many further applications exist where there is the need to transmit control signals or data between a rotor and a stator like in windmills, revolving transfer machines, bottling plants, packaging machines or placement heads of insertion machines.

A capacitive rotary joint for CT scanners is disclosed in US 5,600,697. A large diameter rotating ring carries a differentially driven strip line guiding a signal along this circumference of the ring. The strip line has copper conductors on a PCB base. This strip line has a bandwidth limited to a few GHz and therefore a data rate limited to a few Gbit/s.

A dielectric cable is disclosed in US 2008/036558 A1. A hybrid plastic waveguide is disclosed in EP 3203287 A1. A dielectric waveguide using powdered material is disclosed in US 448004350. A tiltable waveguide member using a ball and socket configuration is disclosed in US 9,871,283 B1. DE 102015105657 A1 discloses a connector for dielectric waveguides.

EP1729646 discloses a datalink based on a dielectric Waveguide. The couplers for coupling signals into and out of the waveguide are comparatively complex.EP 3309898 A1 discloses an antenna module for millimeter-wave communication which combines transmit and receive signals to a transceiver and couples them into a dielectric waveguide by an antenna.

US 2007/0063785 A1 discloses a rotating data transmission device including a dielectric waveguide which is split into at least two segments having approximately the same length.

US 4,692,721 A discloses a dielectric rotary coupler having a dielectric waveguide with a rectangular cross-section to couple a carrier microwave FM-modulated signal.

US 2017/332991 A1 discloses a transmission system for the contactless transmission of an electrical and/or electromagnetic signal.

EP 2 932 901 A1 discloses a rotary joint for multichannel high speed data transmission where the rotating part comprises N input channels for receiving data of N data channels, a routing means for routing the signals of the input channels to N+1 transmission lines by delay units and transmitter amplifiers. The corresponding stationary part comprises N receiving couplers connected to N receiving amplifiers for delivering the signal to N output channels.

### Summary of the invention

The problem to be solved by the invention is to provide a contactless datalink for transmission of data between rotating parts in a mm wave band. The device should be simple, easy to install, easy to maintain and cost - efficient.

Solutions of the problem are described in the independent claims. The dependent claims relate to further improvements of the invention.

In an embodiment, a contactless datalink for transmission of data between a rotating part and a stationary part comprises a ring shaped dielectric waveguide which is split into two components, preferably two identical and/or symmetrical components and forming a disc configuration. One component, for example a first component is mounted at the stationary part of a rotating device, and a second component is mounted at the rotating part. The dielectric waveguide may have a round cross section as shown or an oval or rectangular cross section. In an embodiment, the rotating and stationary parts may be exchanged.

There is a gap between the two components in a plane, preferably orthogonal to the rotation axis, forming the center of rotation. The ring may be split cylindrically and parallel to the rotation axis to form a drum configuration. The stationary waveguide component and the rotating waveguide component are in close proximity with each other such that signals are coupled between the stationary and rotating waveguides. The ring-shaped dielectric waveguide has a center axis aligned with the axis of rotation between the rotating and the stationary part. The rotating and the stationary part are rotatable around said axis of rotation. Here the term of identical and/or symmetrical components preferably relates to the structure of these components, for example cross section or ring diameter. The first dielectric waveguide component comprises at least one first rotating waveguide section, having two ends wherein one end is connected to at least one of a transmitter, a receiver, or an absorber and the other end is connected to at least one of a transmitter, a receiver, or an absorber. The second dielectric waveguide component comprises at least one first stationary waveguide section, having two ends wherein one end is connected to at least one of a transmitter, a receiver, or an absorber and the other end is connected to at least one of a transmitter, a receiver, or an absorber.

The width of the gap is preferably dimensioned in the range of 1/10th to 1/6th of a wavelength (of a signal transmitted through the dielectric waveguide) in air and may have any size between nearly zero and 1/4th of a wavelength in air. There may be changes and variations in this range for example caused by mechanical tolerances. Later, reference will be made to these waveguide components as waveguides.

The dielectric wave guide may have a cladding comprising a dielectric with lower dielectric constant than the core. The core or dielectric waveguide may have a round cross section as shown or an oval or rectangular cross section.

The split waveguide preferably is held by a mechanical support or cladding that has a lower dielectric constant than the waveguide itself, and which for example may comprise a plastic foam or a different material.

The waveguide preferably has a low dielectric constant and low losses in the frequency range of and above 60GHz (Millimeter Wave range which is defined as having a wavelength between 1 mm and 10 mm corresponding to a frequency range of 30 GHz to 300 GHz. This frequency band is called EHF for Extremely High Frequency). Preferred materials are plastics with a low relative dielectric constant (1.5 to 3.5) and low losses like Polytetrafluorethylene (PTFE) and (PP) Polypropylene, Polyethylene or Polystyrene, chlorinated Polyethylene (CPE), Polyether ether ketone (PEEK), Polyphenylene sulfide (PPS), Cyclo-olefin polymer (COP) or Polyimide or combinations thereof. In an embodiment, the waveguide made from solid plastic. It may also be filled with ceramics or made as foam to adjust the dielectric constant.

In an embodiment, which may be based on a waveguide structure as described above, a stationary waveguide (or waveguide component) forming a circle, preferably both in a same plane parallel to the plane mentioned above and around the axis of rotation is split into two sections, and preferably having the same length. A second waveguide component at the second part which may be the rotating part, comprises also a first section and a second section. Preferably, the second waveguide component is the symmetrical to the first waveguide component and mounted close to the first waveguide component to form a narrow gap thereto. The stationary waveguide component and the rotating waveguide component are in close proximity which each other such that signals are coupled between the stationary and rotating waveguides, preferably in both directions - from the stationary waveguide component to the rotating waveguide component and from the rotating waveguide component to the stationary waveguide component. Preferably all waveguide sections have about the same length, and therefore cover about half the circumference of their part, corresponding to an angle of approximately 180 degrees. The first stationary waveguide section and the second stationary waveguide section are connected with two ends which are closed together to a 3dB coupler which is further coupled to a receiver. The 3dB coupler combines the signals of both stationary waveguide sections and forwards these to the receiver. At the opposing ends, there may be absorbers. The rotating waveguide sections and are connected with ends close together to a 3dB coupler which is further connected to a transmitter. The signals from the transmitter are forwarded to the 3dB coupler which splits the signal into two equal signals which are fed into each of the rotating waveguide sections and. The opposing ends of the rotating waveguide sections may also be also terminated by absorbers.

In a further embodiment, the stationary waveguide sections and are connected at positions close to each other with a transmitter and a receiver, such that the first stationary waveguide section is connected to receiver, and the second stationary waveguide section is connected to a transmitter. The first rotating section is connected at a first end with a first 3dB coupler, which is further connected to a receiver. The second end is connected to a second 3dB coupler which is further connected to a transmitter. Further, in a clockwise direction, the first end of the second rotating waveguide section is connected to the first 3dB coupler, whereas the second end of the second rotating waveguide is connected to the second 3dB coupler. This allows a transmission without change in phase. The angular gap prevents cross-coupling. The term of "close to" may refer to the ends of dielectric waveguide sections which are in proximity to each other.

In a further embodiment several identical or different of the above described embodiments might be installed in parallel or antiparallel to increase the data transmission capacity in a given application.

In an embodiment, a contactless datalink is configured to operate at an operating frequency within the EHF band. It may also be configured to operate at an operating frequency between 2.4GHz and 30GHz.

There are further embodiments having different combinations of waveguides, waveguide sections, transmitters and receivers. Examples of these embodiments are described in the drawings sections in more detail.

### Description of Drawings

In the following the invention will be described by way of example, without limitation of the general inventive concept, on examples of embodiment with reference to the drawings

The examples encompassed by the scope of the appended claims are those of figures 1-6.

The examples of figures 7-11 do not fall into the scope of the appended claims, and are useful for understanding the state of the art.
Figure 1a, 1b, 1c show different modifications of a first embodiment of a dielectric waveguide.
Figure 2 shows a basic embodiment of a single channel unidirectional data communication.
Figure 3 shows a further embodiment of a single channel bidirectional data communication.
Figure 4 shows a further embodiment of a multichannel bidirectional data communication.
Figure 5 shows a further embodiment of a multichannel bidirectional data communication.
Figure 6 shows a further embodiment.
Figure 7 shows a basic concept of a further dielectric waveguide in a sectional view.
Figure 8 shows schematically a CT (Computed Tomography) scanner gantry.
Figure 9 shows a further bidirectional embodiment employing transceivers.
Figure 10 shows a further bidirectional embodiment employing transceivers.
Figure 11 shows a further bidirectional embodiment employing transceivers.

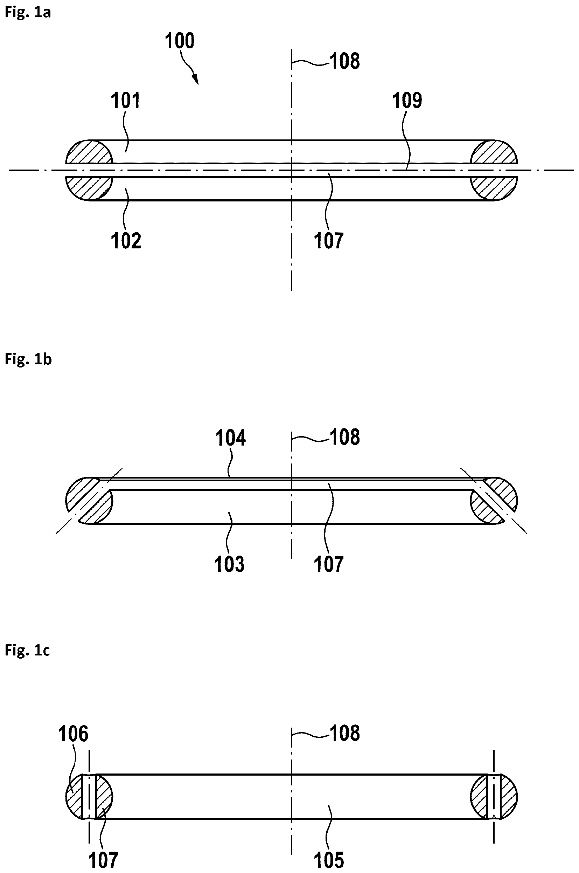

In Figure 1a, a first embodiment is shown. A dielectric waveguide 100 comprises two waveguide components, preferably two identical and/or symmetrical components 101 and 102, forming a disc configuration. One component, for example. a first component 101 is mounted at the stationary part, and a second component 102 is mounted at the rotating part. The dielectric waveguide components may together have a round cross section as shown or an oval or rectangular cross section. In an embodiment, the rotating and stationary parts may be exchanged. There is a gap 107 between the two component in a plane 109 preferably orthogonal to the rotation axis 108.

As shown in Fig. 1b, in a further embodiment, the ring comprising a first dielectric waveguide component 103 and a second dielectric waveguide component 104 may be split under any angle, like a slanted angle or a 45 degrees angle which is between the above-mentioned embodiments. In another embodiment, the ring comprising a first dielectric waveguide component 105 and a second dielectric waveguide component 106 may be split (forming the two components), as shown in Fig. 1c, cylindrically and parallel to the rotation axis to form a drum configuration.

Any configuration allows coupling of signals from either side to the other side.

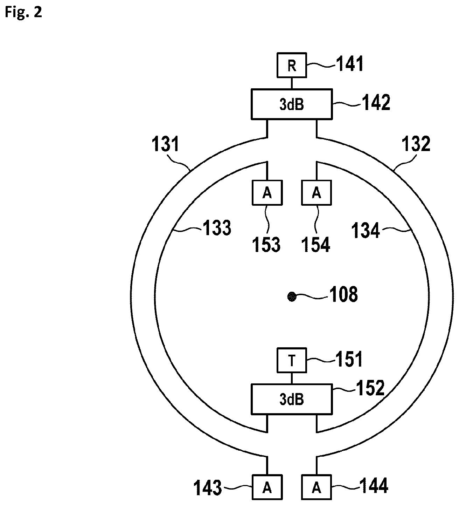

In Figure 2, a basic embodiment is shown which may be based on a waveguide structure of the first embodiment. A stationary waveguide 131, 132 comprising a first section 131 and a second section 132 is at a first part. The rotating part's waveguide comprises also a first section 133 and a second section 134. The stationary waveguides and the rotating waveguides are in close contact which each other such that signals are coupled between the stationary and rotating waveguides, preferably in both directions - from the stationary waveguide to the rotating waveguide and from the rotating waveguides to the stationary waveguides. Preferably all waveguide sections have about the same length, and therefore cover about half the circumference of their part, corresponding to an angle of approximately 180 degrees. The waveguide sections preferably have two ends. The first stationary waveguide section 131 and the second stationary waveguide section 132 are connected with two ends which are closed together to a 3dB coupler 142 which is further coupled to a receiver 141. The coupler combines the signals of both stationary waveguide sections and forwards these to the receiver. At the opposing ends, there are absorbers 143 and 144. The rotating waveguide sections 133 and 134 are connected with ends close together to a 3dB coupler 152 which is further connected to a transmitter 151. The signals from the transmitter 151 are forwarded to the 3dB coupler 152 which splits the signal into two equal signals which are fed into each of the rotating waveguide sections 133 and 134. The opposing ends of the rotating waveguide sections are also terminated by absorbers 153 and 154.

Preferably, the transmitter 151 generates and modulates rf signals in the range of and above 60GHz and couples a wave into the dielectric waveguide where it propagates towards a receiver 141.

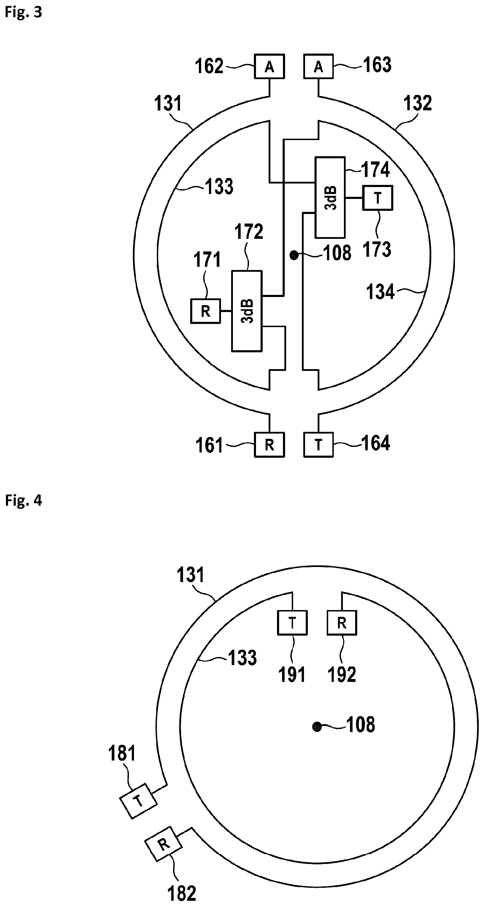

In Figure 3, a further embodiment is shown. Here, the stationary waveguide sections 131 and 132 are connected at positions close to each other with a transmitter 164 and a receiver 161, such that the first stationary waveguide section 131 is connected to receiver 161, and the second stationary waveguide section 132 is connected to transmitter 164. The first rotating section 133 is connected at a first end with a first 3dB coupler 172, which is further connected to a receiver 171. The second end is connected to a second 3dB coupler 174 which is further connected to a transmitter 173. Further, in a clockwise direction, the first end of the second rotating waveguide section 134 is connected to the first 3dB coupler 172, whereas the second end of the second rotating waveguide 134 is connected to the second 3dB coupler 174. This allows a transmission without change in phase. The angular gap prevents cross-coupling between the ends of the rotating waveguide sections 133 and 134.

In Figure 4, a further embodiment is shown with only one first waveguide 131 and one second waveguide 133. First waveguide 131 is connected at one end to a receiver 182 and at the opposing end to a transmitter 181. The second waveguide 133 is connected at a first to a receiver 192 and at the opposing end to a transmitter 191. The link is built such that the arrangement of transmitter and receiver is in opposing directions. A gap between receiver and transmitter causes a high attenuation between receiver and transmitter.

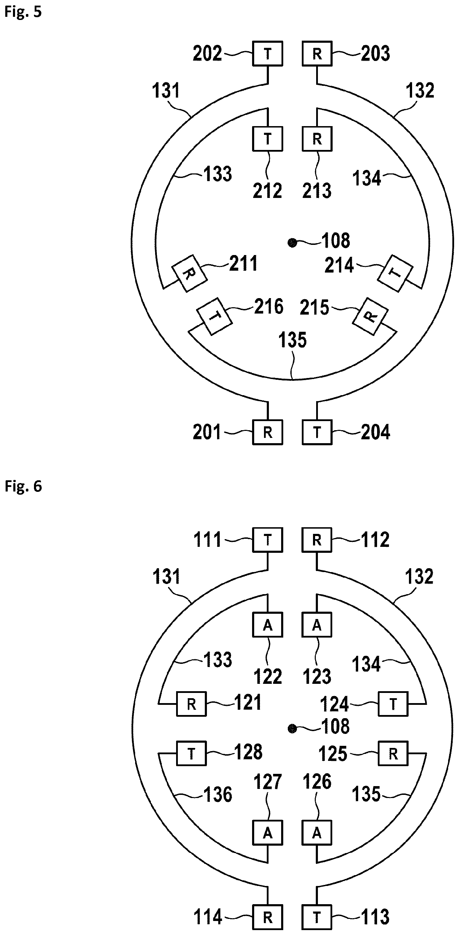

Alternatively both ends of the first stationary waveguide section 131 can be joined to a single waveguide at the end and connected to a transceiver combining receiver 182 and transmitter 181.Also both ends of the first rotating waveguide section 133 can be joined to a single waveguide at the end and connected to a transceiver combining receiver 192 and transmitter 191 In Figure 5, a further embodiment is shown wherein the first waveguide comprises a first stationary waveguide section 131 and a second stationary waveguide section 132 which have the same length and therefore cover an angle of about 180 degrees. The rotating waveguide comprises of a first rotating waveguide section 133, a second rotating waveguide section 134, and a third rotating waveguide section 135 which all have about the same size, and therefore cover an angle of about 120 degrees. The first stationary waveguide section 131 is connected at a first end to a receiver 201 and to a transmitter 202 at the opposing end. Clockwise next to this end, the first end of the second stationary waveguide section 132 is connected to a receiver 203, and the opposing end to a transmitter 204. Therefore, transmitters and receivers are alternatingly connected at the ends of the first waveguide sections, wherein a receiver is followed by a transmitter in a clockwise view. At the rotating waveguide sections, there is basically the same arrangement, such that a transmitter is followed by a receiver in a clockwise view on each of the three sections. Therefore, the first rotating waveguide section 133 has a receiver 211 at a first end and opposing thereto a transmitter 212. Next to this, the second rotating waveguide section 134 has a receiver 213 at its first end and a transmitter 214 at its second end. This is followed by the third rotating waveguide section having a receiver 215 at its first end and a transmitter 216 at its second end. The second end is close to the first end of the first rotating waveguide section 133. It is obvious, that the sequence of transmitters and receivers may be reversed.

Also, one end of the first stationary waveguide section 131 and 132 can be joined to a single waveguide at the end and connected to a transceiver combining receiver 203 and transmitter 202 and another transceiver combining receiver 201 and transmitter 204. Also, receiver 213 and transmitter 212, receiver 215 and transmitter 214 and receiver 211 and transmitter 216 can be paired to transceivers.

In Figure 6, a further embodiment is shown. A first part, for example the stationary part, may bear stationary waveguide sections 131, 132. A second part, for example a stationary part, may bear stationary waveguide sections 133, 134, 135, 136 which all have about the same size, and therefore cover an angle of about 90 degrees each. The stationary waveguides 131, 132 have receivers 112, 114 at their first ends and transmitters 111, 113 at their second ends. Accordingly, in the clockwise direction, first waveguide 131 has a receiver 114 at its first end and a transmitter 111 at its second end, whereas the first waveguide 132 has a receiver 112 at its first end and a transmitter 113 at its second end.

The rotating waveguides have either a transmitter or a receiver at one end, and an absorber at the opposing end. Here, the first rotating waveguide 133 has in clockwise direction at its first end a receiver 121 and an absorber 122 opposing thereto. The second rotating waveguide section 134 has an absorber 123 at an end close to the absorber 122 and a transmitter 124 opposing thereto. The third rotating waveguide section 135 has a receiver 125 close to the transmitter 124 and opposing thereto an absorber 126. The fourth rotating waveguide section 136 has an absorber 127 close to the absorber 1226and opposing thereto a transmitter 128.

All the embodiments above show exemplary embodiments of the invention. It is obvious, that the function of the rotating and the stationary part may be exchanged. Furthermore, the orientation of the transmitters or receivers may be exchanged with respect to the waveguides. In any case, in each of the embodiments, each of the transmitters may be exchanged by a receiver, and each of the receivers may be exchanged by a transmitter.

All transmitters and receivers shown in figures 4 to 6 as well as receiver 161 and transmitter 164 in Fig 3 may be combined to a transceiver when both half waveguides are combined to a full waveguide in direct vicinity of the transceivers.

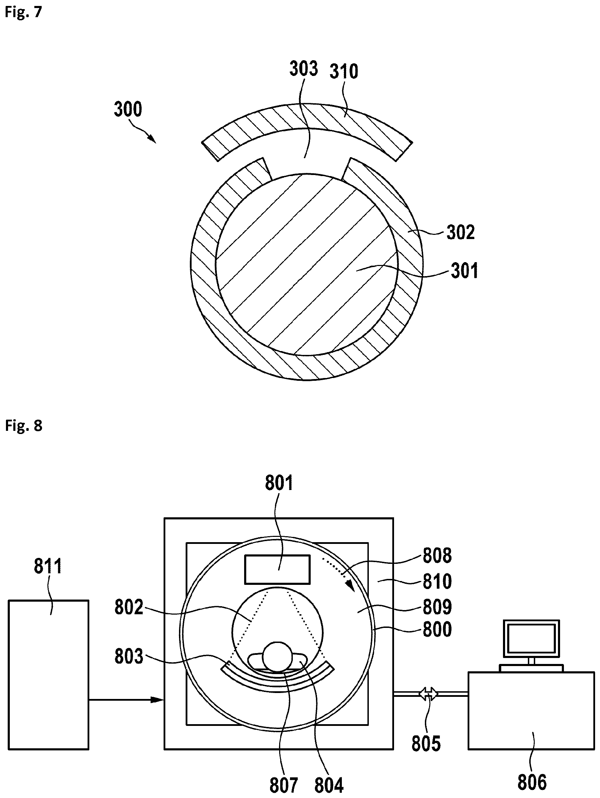

In Figure 7, a basic concept of a further dielectric waveguide 300 in a sectional view is shown. The waveguide has a dielectric core 301 and a metallic shield 302 which has a gap 303. For coupling a signal, there may be a pickup 310 which preferably has only a short length of few millimeters or few centimeters, compared to the total length of the dielectric waveguide which may be in the range of some meters. It is preferred, if energy is only radiated out of the gap at a position where the pickup is located, as there the impedance should be matched.

Figure 8 shows schematically a CT (Computed Tomography) scanner gantry. The stationary part is suspended within and part of a massive frame 810. The rotating part 809 of the gantry is rotatably mounted with respect to the stationary part and rotates along the rotation direction 808. It supports an X - ray tube 801 for generating an X-ray beam 802 that radiates through a patient 804 lying on a table 807 and which is intercepted by a detector 803 and converted to electrical signals and imaging data thereof. Electrical power from power supply unit 811 may be transmitted by a slipring (not shown) to the rotating part. The data obtained by the detector 803 are transmitted via contactless rotary joint 800 to an evaluation unit 806 by means of a data bus or network 805.

In the description of the next figures the closed loop dielectric waveguide 400 is abbreviated to waveguide 400 for better readability.

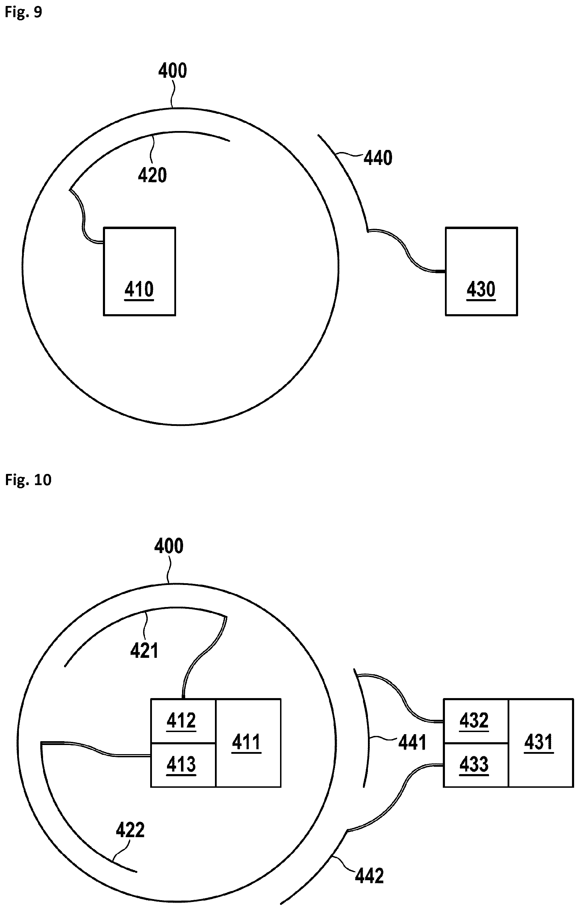

In Figure 9, a basic embodiment is shown which may be based on a standard dielectric waveguide structure 400 forming a closed loop and including one part that is formed to a circle where both ends are glued or welded together or that is formed as one part. This circular dielectric waveguide might be fixed to the stator (stationary) or fixed to the rotor (rotating). The cross section of the waveguide 400 might be shaped circular, elliptic, oval or rectangular or of any other shape suited to guide a wave.

Also, this waveguide 400 might be identical to one waveguide section 101 as described in Fig. 1, that means it is only one section of a dielectric waveguide split into two sections where either section can guide a wave but also the combined sections.

Two couplers (420, 440) formed of a standard dielectric waveguide as described may be used to couple a wave in and/or out of the waveguide 400. These couplers are kept in close distance preferably dimensioned in the range of 1/6^{th} to 1/10^{th} of a wavelength in air, the distance may vary because of mechanical tolerances between near zero and up to 1/4^{th} of a wavelength. The couplers preferably are mounted as circular segments having the same center point as the waveguide 400. The length of each of the two couplers preferably are identical and are shorter than half of the total circumference.

The waveguide 400 might also rotate in a different speed than the rotating coupler. Since in a preferred configuration the waveguide 400 is either rotating or stationary, one of the couplers might be mounted in a fixed position and distance to the waveguide 400. The rotating coupler 440 is mounted in such a way that it cannot collide with the stationary coupler during rotation. This means that one coupler (e.g. the stationary coupler 420 as in this figure) can be mounted on the inner side of the waveguide 400, the other coupler (e.g. the rotating coupler 440 as in this figure) on the outer diameter. Alternatively, the couplers can be mounted before or behind the waveguide 400 or at any angular disposition where a collision is avoided.

One or both of the couplers (420, 440) can also be part of a waveguide section 102 as described in Fig. 1, that means that the coupler is formed of a part of the other section of a waveguide split into two sections where either section can guide a wave but also the combined sections. Preferably this is the rotating coupler 440 when the waveguide 400 is stationary.

The transceivers 410, 430 include a transmitter and receiver that are connected to one port of the transceiver for wireless transmission and reception of. This port can also be used for "wired" communication through a dielectric waveguide if the port is coupled to a dielectric waveguide capable to guide the wave. The stationary transceiver (410) having one port to transmit and receive signals wireless and is connected to the stationary coupler (420) either by a connecting dielectric waveguide or by directly coupling a wave into the coupler e.g. by an antennaor by a coaxial cable connecting the transceiver to an antenna which then couples the signal into the coupler. As the stationary transceiver (410) also the rotating transceiver (430) is having one port to transmit and receive signals wireless and is connected to the stationary coupler (420) The waveguide 400 and couplers (420, 440) which are also formed of waveguides as described, are in close contact which each other such that signals are coupled between the stationary and rotating coupler with the waveguide 400 in between, preferably in both directions.

In the configuration described in this fig. 9 data transmitted up (from stationary transceiver 410 to rotary transceiver 430) are propagating as waves in the waveguide 400 in clockwise direction, the data transmitted down (from rotary transceiver 430 to stationary transceiver 410) are propagating in the contrary direction. Preferably the waveguide 400 has an attenuation low enough to allow communication between the both transceivers during a first round of the waves travelling but high enough not to distort the receiver after the first round is completed

Preferably, the stationary transceiver (410) generates and modulates rf signals in the range of and above 60GHz and couples a wave into the dielectric waveguide where it propagates towards the rotary transceiver (430) and vice versa. The technology can also be used with all carrier frequencies that can be coupled into dielectric waveguides, e.g. operating at 2.4GHz or 5GHz or any frequency above that but below optical frequencies.

Both transceivers have a another wired port that is not shown which is electrically coupled to transmit and receive data via a bus. The waveguide 400 and the couplers (410, 430) form a contactless rotary joint for bidirectional data transmission. In case of the CT gantry shown and described in fig. 8. The data obtained by the detector 803 of fig. 8 is transmitted via the rotating transceiver 430 through the contactless rotary joint described in fig. 9 to the stationary transceiver which is connected by bus (not shown) to the stationary evaluation unit 806 of fig. 8 where the data are received, control data are transmitted in the opposite direction.

Fig. 10 basically shows the same principle setup of rotating couplers and waveguide 400 as in fig. 9. The difference is that a multiport transceiver 411 with at least a first stationary port 412 and at least a second stationary port 413 is used that optimizes transmission quality and/or data rate by controlling carrier frequency, phase, amplitude and modulation parameters of each rf port to separately match changing attenuation and phase during the rotation and to avoid crosstalk between the channels.

In the configuration described data transmitted up (from stationary multiport transceiver 411 to rotary multiport transceiver 431) are propagating as modulated waves in the waveguide in clockwise direction, the data transmitted down (from rotary multiport transceiver 431 to stationary transceiver 411) are propagating in the contrary direction. Preferably the waveguide 400 has an attenuation low enough to allow communication between the both transceivers during a first half round of the waves travelling but high enough not to distort the receiver after the first round is completed

In this configuration, the first stationary port 412 and the second stationary port 413 of the stationary multiport transceiver 411 communicate via first stationary coupler 421, second stationary coupler 422, waveguide 400 and first rotating coupler 441, second rotating coupler 442 with the first rotating port 432 and the second rotating port 433of the rotating multiport transceiver 431. The transceivers select as inputs the rf port with the best communication quality, which changes during rotation, typically determined by bit error rate. To optimize the transmission by lowering the crosstalk between the channels the first and second rotating couplers 441,442 may be positioned approximately 180 degrees apart of each other. The same applies for the first and second rotating couplers 441,442 which preferably are positioned approximately 180 degrees apart of each other. With n (n = 1,2,3,4,5, ...) ports the displacement of the couplers should be approximately 360 degrees divided by n. The fig. 10 shows a displacement of approximately 90degrees.

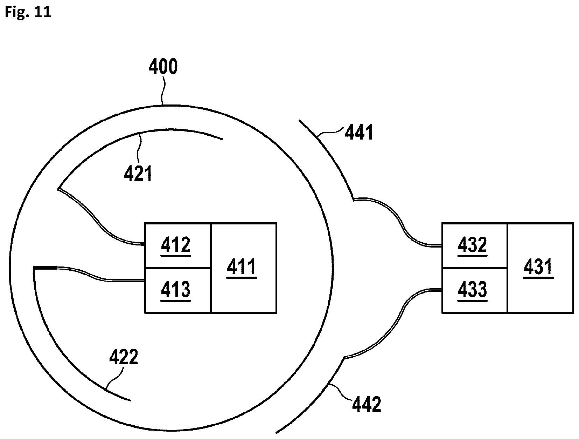

Preferably the transceivers are employing the MIMO (multiple in - multiple out) technology as e.g. defined in recent substandards of IEEE-802.11 -(WiFi/WLAN-Standard) e.g. IEEE-802.11n and newer substandards-(WiFi/WLAN-Standard). Another implementation into a widely used commercial standard is LTE at e.g. 2.6GHz. These standards for wireless communication optimize by allowing any port of one transceiver to communicate with any port of the corresponding transceiver. Fig. 11 is similar to Fig. 10.

In the configuration port the first stationary port 412 of the stationary multiport transceiver 411 communicates via stationary coupler 421, waveguide 400 and rotating coupler 441 with port the first rotating port 432 of the rotating multiport transceiver 431, the second stationary port 413 of the stationary multiport transceiver 411 communicates via stationary coupler 421, waveguide 400 and rotating coupler 441 with the second rotating port 433 of the rotating multiport transceiver 431.

Here preferably the closed loop waveguide 400 has an attenuation low enough to allow communication between the both transceivers during a first half round of the waves travelling but high enough not to distort the receiver after the first round is completed. To optimize the transmission the first and second rotating couplers 441,442 may be positioned approximately 180 degrees apart of each other. The same applies for the first and second rotating couplers 441,442 which may be positioned approximately 180 degrees apart of each other. With n (n = 1,2,3,4,5, ...) ports the displacement of the couplers should be approximately 360degress divided by n. The fig. 11 shows a displacement of approximately 90degrees.

### List of reference numerals

- 100: ring shaped dielectric waveguide
- 101: first dielectric waveguide component
- 102: second dielectric waveguide component
- 103: first dielectric waveguide component
- 104: second dielectric waveguide component
- 105: first dielectric waveguide component
- 106: second dielectric waveguide component
- 107: gap
- 108: rotation axis
- 109: plane
- 111: transmitter
- 112: receivers
- 113: transmitter
- 114: receivers
- 121: receiver
- 122: absorber
- 123: absorber
- 124: transmitter
- 125: receiver
- 126: absorber
- 127: absorber
- 128: transmitter
- 131: first stationary waveguide section
- 132: second stationary waveguide section
- 133: first rotating waveguide section
- 134: second rotating waveguide section
- 135: third rotating waveguide section
- 136: fourth rotating waveguide section
- 141: receiver
- 142: 3dB coupler
- 143: absorber
- 144: absorber
- 151: transmitter
- 152: 3dB coupler
- 153: absorber
- 154: absorber
- 161: transmitter
- 164: receiver
- 171: receiver
- 172: first 3dB coupler
- 173: transmitter
- 174: second 3dB coupler
- 181: transmitter
- 182: receiver
- 191: transmitter
- 192: receiver
- 201: receiver
- 202: transmitter
- 203: receiver
- 204: transmitter
- 211: receiver
- 212: transmitter
- 213: receiver
- 214: transmitter
- 215: receiver
- 216: transmitter
- 300: dielectric waveguide
- 301: dielectric core
- 302: metallic shield
- 303: gap
- 310: pickup
- 400: closed loop dielectric waveguide
- 410: stationary transceiver
- 411: stationary multiport transceiver
- 412: first stationary port
- 413: second stationary port
- 420: stationary coupler
- 421: first stationary coupler
- 422: second stationary coupler
- 430: rotating transceiver
- 431: rotating multiport transceiver
- 432: first rotating port
- 433: second rotating port
- 440: rotating coupler
- 441: first rotating coupler
- 442: second rotating coupler
- 800: contactless rotary joint
- 801: x-ray tube
- 802: x-ray beam
- 803: x-ray detector
- 804: patient
- 805: network
- 806: evaluation unit
- 807: patient table
- 808: rotation direction
- 809: rotating part
- 810: frame
- 811: power supply unit

## Claims

1. A contactless datalink for transmission of data between a rotating part and a stationary part, rotatable around an axis of rotation (108) the datalink comprising a ring shaped dielectric waveguide (100), which is split into two components,
wherein a first dielectric waveguide component (101) is mounted at the rotating part and a second dielectric waveguide component (102) is mounted at the stationary part, the ring shaped dielectric waveguide (100) having a center axis aligned with the axis of rotation (108),
the ring shaped dielectric waveguide (100) having a gap (107) between the first dielectric waveguide component (101) and the second dielectric waveguide component (102),
the stationary waveguide component and the rotating waveguide component are in close proximity with each other such that signals are coupled
between the stationary and rotating waveguides,
the first dielectric waveguide component (101) comprises at least one first rotating waveguide section (133, 134, 135, 136), having two ends wherein one end is connected to at least one of a transmitter (111, 113, 124,128, 151, 161, 173, 181, 191, 202, 204, 212, 214, 216), a receiver (112, 114, 125, 141, 164,171, 182, 192, 201, 203, 211, 213, 215), or an absorber (122,123, 126, 127, 143, 144, 153, 154) and the other end is connected to at least one of a transmitter (111, 113, 124,128, 151, 161, 173, 181, 191, 202, 204, 212, 214, 216), a receiver (112, 114, 125, 141, 164,171, 182, 192, 201, 203, 211, 213, 215), or an absorber (122, 123, 126, 127, 143, 144, 153, 154), the second dielectric waveguide component (102) comprises at least one first stationary waveguide section (131, 132), having two ends wherein one end is connected to at least one of a transmitter (111, 113, 124,128, 151, 161, 173, 181, 191, 202, 204, 212, 214, 216), a receiver (112, 114, 125, 141, 164,171, 182, 192, 201, 203, 211, 213, 215), or an absorber (122,123, 126, 127, 143, 144, 153, 154) and the other end is connected to at least one of a transmitter (111, 113, 124,128, 151, 161, 173, 181, 191, 202, 204, 212, 214, 216), a receiver (112, 114, 125, 141, 164,171, 182, 192, 201, 203, 211, 213, 215), or an absorber (122,123, 126, 127, 143, 144, 153, 154).

2. Contactless datalink according to claim 1,
wherein
the first dielectric waveguide component (101) comprises a first rotating waveguide section (133) and a second rotating waveguide section (134), wherein first ends of the sections which are close together are connected by a 3dB coupler (152) to a transmitter and the other ends are terminated by absorbers (153, 154); and
the second dielectric waveguide component (102) comprises a first stationary waveguide section (131) and a second stationary waveguide section (132), wherein first ends of the sections which are close together are connected by a 3dB coupler (142) to a transmitter and the other ends are terminated by absorbers (143, 144).

3. Contactless datalink according to claim 1,
wherein
the first dielectric waveguide component (101) comprises a first rotating waveguide section (133) and a second rotating waveguide section (134), wherein first ends of the sections are close together and second ends of the sections are close together;
the first end of the first rotating waveguide section (133) and the second end of the second rotating waveguide section (134) are connected by a first 3dB coupler (174) to a transmitter (173); and
the second end of the first rotating waveguide section (133) and the first end of the second rotating waveguide section (134) are connected by a second 3dB coupler (172) to a receiver (171); and
the second dielectric waveguide component (102) comprises a first stationary waveguide section (131) and a second stationary waveguide section (132), wherein first ends of the sections are close together and second ends of the sections are close together;
the first end of the first stationary waveguide section (131) is connected to a receiver (161) and the first end of the second stationary waveguide section (132) is connected to a transmitter (164); and
the second ends are terminated by absorbers (143, 144).

4. Contactless datalink according to claim 1,
wherein
the first dielectric waveguide component (101) comprises a rotating waveguide section (133), wherein one end of the section is connected to a transmitter (191) and the other end is connected to a receiver (192); and the second dielectric waveguide component (102) comprises a stationary waveguide section (131) wherein one end of the section is connected to a transmitter (181) and the other end is connected to a receiver (182).

5. Contactless datalink according to any of the previous claims,
wherein
the first dielectric waveguide section is coupled to a transmitter and the second dielectric waveguide section is coupled to a receiver.

6. Contactless datalink according to any of the previous claims,
wherein
the first dielectric waveguide component (101) has an identical structure to the second dielectric waveguide component (102).

7. Contactless datalink according to any of the previous claims,
wherein
the width of the gap (107) is in a range of 1/10th to 1/6th of a wavelength in air of a signal to be transmitted.

8. Contactless datalink according to any of the previous claims,
wherein
the at least one first rotating waveguide sections (133, 134, 135, 136) have the same length and/or
the at least one first stationary waveguide sections (131, 132) have the same length.

9. A contactless datalink according to any of the preceding claims configured to operate at an operating frequency within the EHF, Extremely High Frequency, band.

10. A contactless datalink according to any of claims 1 to 8 configured to operate at an operating frequency between 2.4GHz and 30GHz.

## Patentansprüche

1. Eine kontaktlose Datenverbindung zur Übertragung von Daten zwischen einem rotierenden Teil und einem stationären Teil, welche um eine Rotationsachse (108) drehbar ist, wobei die Datenverbindung einen ringförmigen dielektrischen Wellenleiter (100) umfasst, welcher in zwei Komponenten gesplittet ist,
wobei
eine erste dielektrische Wellenleiterkomponente (101) am rotierenden Teil montiert ist und eine zweite dielektrische Wellenleiterkomponente (102) am stationären Teil montiert ist,
wobei der ringförmige dielektrische Wellenleiter (100) eine Mittelachse hat, welche mit der Rotationsachse (108) ausgerichtet ist,
wobei der ringförmige dielektrische Wellenleiter (100) einen Spalt (107) zwischen der ersten dielektrischen Wellenleiterkomponente (101) und der zweiten dielektrischen Wellenleiterkomponente (102) hat,
wobei die stationäre Wellenleiterkomponente und die rotierende Wellenleiterkomponente in direkter Nähe zueinander sind, so dass Signale zwischen den stationären und den rotierenden Wellenleitern gekoppelt werden,
wobei die erste elektrische Wellenleiterkomponente (101) mindestens einen ersten rotierenden Wellenleiterabschnitt (133, 134, 135, 136) mit zwei Enden umfasst, wobei ein Ende mit mindestens einem von einem Sender (111, 113, 124, 128, 151, 161, 173, 181, 191, 202, 204, 212, 214, 216), einen Empfänger (112, 114, 125, 141, 164, 171, 182, 192, 201, 203, 211, 213, 215) oder einem Absorber (122, 123, 126, 127, 143, 144, 153, 154) verbunden ist und das andere Ende ist mit mindestens einem von einem Sender (111, 113, 124, 128, 151, 161, 173, 181, 191, 202, 204, 212, 214, 216), einem Empfänger (112, 114, 125, 141, 164, 171, 182, 192, 201, 203, 211, 213, 215) oder einem Absorber (122, 123, 126, 127, 143, 144, 153, 154) verbunden ist, wobei die zweite dielektrische Wellenleiterkomponente (102) mindestens einen ersten stationären Wellenleiterabschnitt (131, 132) mit zwei Enden umfasst, wobei ein Ende mit mindestens einem von einem Sender (111, 113, 124, 128, 151, 161, 173, 181, 191, 202, 204, 212, 214, 216), einem Empfänger (112, 114, 125, 141, 164, 171, 182, 192, 201, 203, 211, 213, 215) oder einem Absorber (122, 123, 126, 127, 143, 144, 153, 154) verbunden ist und das andere mit mindestens einem von einem Sender (111, 113, 124, 128, 151, 161, 173, 181, 191, 202, 204, 212, 214, 216), einem Empfänger (112, 114, 125, 141, 164, 171, 182, 192, 201, 203, 211, 213, 215) oder einem Absorber (122, 123, 126, 127, 143, 144, 153, 154) verbunden ist.

2. Kontaktlose Datenverbindung nach Anspruch 1,
wobei die erste elektrische Wellenleiterkomponente (101) einen ersten rotierenden Wellenleiterabschnitt (133) und einen zweiten rotierenden Wellenleiterabschnitt (134) umfasst, wobei erste Enden der Abschnitte, welche nah beinander sind, durch einen 3dB-Koppler (152) mit einem Sender verbunden sind und die anderen Enden durch Absorber (153, 154) abgeschlossen sind; und
die zweite elektrische Wellenleiterkomponente (102) einen ersten stationären Wellenleiterabschnitt (131) und einen zweiten stationären Wellenleiterabschnitt (132) umfasst, wobei erste Enden der Abschnitte, welche nah beieinander liegen, durch einen 3dB-Koppler (142) mit einem Sender verbunden und die anderen Enden durch Absorber (143, 144) abgeschlossen sind.

3. Kontaktlose Datenverbindung nach Anspruch 1,
wobei
die erste dielektrische Wellenleiterkomponente (101) einen ersten rotierenden Wellenleiterabschnitt (133) und einen zweiten rotierenden Wellenleiterabschnitt (134) umfasst, wobei erste Enden der Abschnitte nah beieinander und zweite Enden der Abschnitte nah beieinander liegen;
wobei das erste Ende des ersten rotierenden Wellenleiterabschnitts (133) und das zweite Ende des zweiten rotierenden Wellenleiterabschnitts (134) durch einen ersten 3dB-Koppler (174) mit einem Sender (173) verbunden sind; und
das zweite Ende des ersten rotierenden Wellenleiterabschnitts (133) und das erste Ende des zweiten rotierenden Wellenleiterabschnitts (134) durch einen zweiten 3dB-Koppler (172) mit einem Empfänger (171) verbunden sind; und
die zweite dielektrische Wellenleiterkomponente (102) einen ersten stationären Wellenleiterabschnitt (131) und einen zweiten stationären Wellenleiterabschnitt (132) umfasst, wobei erste Enden der Abschnitte nah beieinander liegen und zweite Enden der Abschnitte nah beieinander liegen; wobei das erste Ende des ersten stationären Wellenleiterabschnitts (131) mit einem Empfänger (161) verbunden ist und das erste Ende des zweiten stationären Wellenleiterabschnitts (132) mit einem Sender (164) verbunden ist; und
die zweiten Enden durch Absorber (143, 144) abgeschlossen sind.

4. Kontaktlose Datenverbindung nach Anspruch 1,
wobei die erste dielektrische Wellenleiterkomponente (101) einen rotierenden Wellenleiterabschnitt (133) umfasst, wobei ein Ende des Abschnitts mit einem Sender (191) verbunden ist und das andere Ende mit einem Empfänger (192) verbunden ist; und
die zweite dielektrische Wellenleiterkomponente (102) einen stationären Wellenleiterabschnitt (131) umfasst, wobei ein Ende des Abschnitts mit einem Sender (181) verbunden ist und das andere Ende mit einem Empfänger (182) verbunden ist.

5. Kontaktlose Datenverbindung nach einem der vorhergehenden Ansprüche, wobei
der erste dielektrische Wellenleiterabschnitt mit einem Sender verbunden ist und der zweite dielektrische Wellenleiterabschnitt mit einem Empfänger verbunden ist.

6. Kontaktlose Datenverbindung nach einem der vorhergehenden Ansprüche, wobei
die erste dielektrische Wellenleiterkomponente (101) eine identische Struktur wie die zweite dielektrische Wellenleiterkomponente (102) hat.

7. Kontaktlose Datenverbindung nach einem der vorhergehenden Ansprüche, wobei
die Breite des Spaltes (107) in einem Bereich von 1/10 bis 1/6 einer Wellenlänge in Luft eines zu sendenden Signals liegt.

8. Kontaktlose Datenverbindung nach einem der vorhergehenden Ansprüche, wobei
die mindestens einen ersten rotierenden Wellenleiterabschnitte (133, 134, 135, 136) die gleiche Länge haben und/oder
die mindestens einen ersten stationären Wellenleiterabschnitt (131, 132) die gleiche Länge haben.

9. Eine kontaktlose Datenverbindung nach einem der vorhergehenden Ansprüche, welche konfiguriert ist, um mit einer Betriebsfrequenz innerhalb des EHF (Extremely High Frequency)-Bandes zu arbeiten.

10. Eine kontaktlose Datenverbindung nach einem der Ansprüche 1 bis 8, welche konfiguriert ist, um mit einer Betriebsfrequenz zwischen 2,4 GHz und 30 GHz zu arbeiten.

## Revendications

1. Liaison de données sans contact pour une transmission de données entre une partie rotative et une partie fixe, rotative autour d'un axe de rotation (108) la liaison de données comprenant un guide d'onde diélectrique de forme annulaire (100), qui est divisé en deux composants,
dans laquelle
un premier composant de guide d'onde diélectrique (101) est monté au niveau de la partie rotative et un second composant de guide d'onde diélectrique (102) est monté au niveau de la partie fixe,
le guide d'onde diélectrique de forme annulaire (100) ayant un axe central aligné avec l'axe de rotation (108),
le guide d'onde diélectrique de forme annulaire (100) ayant un écartement (107) entre le premier composant de guide d'onde diélectrique (101) et le second composant de guide d'onde diélectrique (102),
le composant de guide d'onde fixe et le composant de guide d'onde rotatif sont à proximité étroite l'un de l'autre de sorte que des signaux soient couplés entre les guides d'onde fixe et rotatif,
le premier composant de guide d'onde diélectrique (101) comprend au moins une première section de guide d'onde rotatif (133, 134, 135, 136), ayant deux extrémités, dans laquelle une extrémité est connectée à au moins l'un parmi un émetteur (111, 113, 124, 128, 151, 161, 173, 181, 191, 202, 204, 212, 214, 216), un récepteur (112, 114, 125, 141, 164, 171, 182, 192, 201, 203, 211, 213, 215), ou un absorbeur (122, 123, 126, 127, 143, 144, 153, 154) et l'autre extrémité est connectée à au moins l'un parmi un émetteur (111, 113, 124, 128, 151, 161, 173, 181, 191, 202, 204, 212, 214, 216), un récepteur (112, 114, 125, 141, 164, 171, 182, 192, 201, 203, 211, 213, 215), ou un absorbeur (122, 123, 126, 127, 143, 144, 153, 154),
le second composant de guide d'onde diélectrique (102) comprend au moins une première section de guide d'onde fixe (131, 132), ayant deux extrémités, dans laquelle une extrémité est connectée à au moins l'un parmi un émetteur (111, 113, 124, 128, 151, 161, 173, 181, 191, 202, 204, 212, 214, 216), un récepteur (112, 114, 125, 141, 164, 171, 182, 192, 201, 203, 211, 213, 215), ou un absorbeur (122, 123, 126, 127, 143, 144, 153, 154) et l'autre extrémité est connectée à au moins l'un parmi un émetteur (111, 113, 124, 128, 151, 161, 173, 181, 191, 202, 204, 212, 214, 216), un récepteur (112, 114, 125, 141, 164, 171, 182, 192, 201, 203, 211, 213, 215), ou un absorbeur (122, 123, 126, 127, 143, 144, 153, 154).

2. Liaison de données sans contact selon la revendication 1,
dans laquelle
le premier composant de guide d'onde diélectrique (101) comprend une première section de guide d'onde rotatif (133) et une seconde section de guide d'onde rotatif (134),
dans laquelle des premières extrémités des sections qui sont proches l'une de l'autre sont connectées par un coupleur 3dB (152) à un émetteur et les autres extrémités sont terminées par des absorbeurs (153, 154) ;
le second composant de guide d'onde diélectrique (102) comprend une première section de guide d'onde fixe (131) et une seconde section de guide d'onde fixe (132), dans laquelle des premières extrémités des sections qui sont proches l'une de l'autre sont connectées par un coupleur 3dB (142) à un émetteur et les autres extrémités sont terminées par des absorbeurs (143, 144).

3. Liaison de données sans contact selon la revendication 1,
dans laquelle
le premier composant de guide d'onde diélectrique (101) comprend une première section de guide d'onde rotatif (133) et une seconde section de guide d'onde rotatif (134),
dans laquelle des premières extrémités des sections sont proches l'une de l'autre et des secondes extrémités des sections sont proches l'une de l'autre ;
la première extrémité de la première section de guide d'onde rotatif (133) et la seconde extrémité de la seconde section de guide d'onde rotatif (134) sont connectées par un premier coupleur 3dB (174) à un émetteur (173) ; et
la seconde extrémité de la première section de guide d'onde rotatif (133) et la première extrémité de la seconde section de guide d'onde rotatif (134) sont connectées par un second coupleur 3dB (172) à un récepteur (171) ; et
le second composant de guide d'onde diélectrique (102) comprend une première section de guide d'onde fixe (131) et une seconde section de guide d'onde fixe (132), dans laquelle des premières extrémités des sections sont proches l'une de l'autre et des secondes extrémités des sections sont proches l'une de l'autre ;
la première extrémité de la première section de guide d'onde fixe (131) est connectée à un récepteur (161) et la première extrémité de la seconde section de guide d'onde fixe (132) est connectée à un émetteur (164) ; et
les secondes extrémités sont terminées par des absorbeurs (143, 144).

4. Liaison de données sans contact selon la revendication 1,
dans laquelle
le premier composant de guide d'onde diélectrique (101) comprend une section de guide d'onde rotatif (133), dans laquelle une extrémité de la section est connectée à un émetteur (191) et l'autre extrémité est connectée à un récepteur (192) ; et
le second composant de guide d'onde diélectrique (102) comprend une section de guide d'onde fixe (131) dans laquelle une extrémité de la section est connectée à un émetteur (181) et l'autre extrémité est connectée à un récepteur (182).

5. Liaison de données sans contact selon l'une quelconque des revendications précédentes,
dans laquelle
la première section de guide d'onde diélectrique est couplée à un émetteur et la seconde section de guide d'onde diélectrique est couplée à un récepteur.

6. Liaison de données sans contact selon l'une quelconque des revendications précédentes,
dans laquelle
le premier composant de guide d'onde diélectrique (101) a une structure identique au second composant de guide d'onde diélectrique (102).

7. Liaison de données sans contact selon l'une quelconque des revendications précédentes,
dans laquelle
la largeur de l'écartement (107) est dans une plage de 1/10^{e} à 1/6^{e} d'une longueur d'onde dans l'air d'un signal à transmettre.

8. Liaison de données sans contact selon l'une quelconque des revendications précédentes,
dans laquelle
les au moins une première section de guide d'onde rotatif (133, 134, 135, 136) ont la même longueur et/ou
les au moins une première section de guide d'onde fixe (131, 132) ont la même longueur.

9. Liaison de données sans contact selon l'une quelconque des revendications précédentes, configurée pour fonctionner à une fréquence de fonctionnement dans la bande des ondes millimétriques, EHF.

10. Liaison de données sans contact selon l'une quelconque des revendications 1 à 8, configurée pour fonctionner à une fréquence de fonctionnement entre 2,4 GHz et 30 GHz.
